# FASCICULE DE BREVET EUROPEEN

(11) **EP 1 340 515 B1**
(45) Date de publication et mention de la délivrance du brevet: **12.09.2007**
(21) Numéro de dépôt: 03290039.1
(22) Date de dépôt: 08.01.2003
(51) Int. Cl.: A61M 16/12

(54) **Dispositif d'assistance respiratoire**
Vorrichtung zur Atmungsunterstützung
Device for breathing assistance

(30) Priorité: 27.02.2002 FR 0202468
(43) Date de publication de la demande: 03.09.2003
(73) Titulaire: Boussignac, Georges, 92160 Antony (FR); Labrune, Jean-Claude, F-92310 Sèvres (FR)
(72) Inventeur: Boussignac, Georges, 92160 Antony (FR); Labrune, Jean-Claude, F-92310 Sèvres (FR)
(74) Mandataire: Bonnetat, Christian

(56) Documents cités:
- EP-A- 0 390 684
- EP-A- 0 692 273
- WO-A-93/17744
- FR-A- 2 782 925
- US-A- 4 584 998

## Description

La présente invention concerne un dispositif d'assistance respiratoire permettant d'apporter de façon efficace des soins médicamenteux d'urgence à un patient en danger de mort.

Un dispositif d'assistance respiratoire comportant les caractéristiques du préambule de la revendication 1 est connu du document FR-A-2782925.

On sait que, par exemple en cas d'arrêt cardiaque, on cherche à ranimer d'urgence un patient par massage et que, pour augmenter l'efficacité d'un tel massage, on administre audit patient un médicament vasoconstricteur, tel que l'adrénaline. Ce médicament est administré soit par voie intraveineuse, soit à travers un tube endotrachéal. Dans le premier cas, l'arrivée du médicament aux poumons est relativement lente, alors que dans le second, le médicament se disperse et n'atteint pas forcément lesdits poumons.

Par ailleurs, par le document EP-0 390 684, on connaît déjà un dispositif d'assistance respiratoire en forme de tube, qui peut être introduit dans la bouche ou le nez d'un patient et qui, outre le canal principal formé par le tube, comporte au moins un canal auxiliaire, par exemple ménagé dans la paroi dudit tube, permettant l'injection d'un jet de gaz respirable (oxygène, air ou mélange air-oxygène) destiné à la ventilation du patient, ces canaux auxiliaires débouchant dans le canal principal au voisinage de l'extrémité distale de ce dernier.

Pour éviter que les jets de gaz respirable viennent frapper directement la muqueuse du patient sous ventilation, risquant par leur énergie cinétique de traumatiser ladite muqueuse, on prévoit dans ce dispositif connu qu'au moins l'extrémité distale desdits canaux auxiliaires débouchant dans le canal principal soit parallèle à celui-ci et que, en regard de l'orifice distal de chaque canal auxiliaire, soient prévus des moyens de déflexion desdits jets de gaz respirable de ventilation vers l'intérieur dudit canal principal.

Ainsi, les jets de gaz respirable traversant lesdits canaux auxiliaires sont défléchis vers l'axe du canal principal, lorsqu'ils pénètrent dans celui-ci. Des mesures expérimentales ont montré que, en aval desdits moyens de déflexion, il se forme, à l'intérieur dudit canal principal, une zone de pression de forme oblongue prenant naissance aux débouchés desdits canaux auxiliaires dans le canal principal et s'allongeant en direction axiale le long de l'axe dudit canal principal avec réduction progressive de sa section transversale, pour n'occuper que la partie centrale de celui-ci, mais que, en aval de ladite zone de pression élevée, la pression desdits jets de gaz respirable chute et les jets de gaz sortent à faible pression à travers l'orifice distal du tube. L'expérience a également montré, qu'en aval de la sortie distale du tube, la pression est faible et maintenue constante dans tout l'espace respiratoire. Cette pression est dépendante du débit de gaz respirable dans les canaux auxiliaires. Par suite, avec le dispositif d'assistance respiratoire conforme au document ci-dessus, on peut par exemple apporter de l'oxygène ou un mélange air-oxygène directement dans les poumons d'un patient, à hauteur de la carène, et supprimer ainsi l'espace mort qui existe dans les autres sondes connues et qui est d'environ un tiers du volume respiratoire total pour un adulte et environ la moitié pour les nouveau-nés prématurés. La suppression de cet espace mort correspond à une augmentation de performance du cycle respiratoire de plus de 25% dans tous les cas de malades et de près de 50% dans certains cas.

Le dispositif du document EP-A-0 390 684 est donc particulièrement avantageux.

L'objet de la présente invention est de perfectionner ce dispositif connu afin de lui permettre d'administrer des médicaments de façon efficace, non seulement pour résoudre le problème d'administration d'un vasoconstricteur en cas d'urgence cardiaque rappelé ci-dessus, mais encore dans tous les cas où cela est utile.

A cette fin, selon l'invention, le dispositif d'assistance respiratoire comporte les caractéristiques de la revendication 1.

Ainsi, grâce à la présente invention, le fluide médicamenteux, qui peut être gazeux ou liquide, est nébulisé et amené directement sur les poumons du patient sous ventilation tout comme le gaz respirable de ventilation, alors que lesdits poumons sont gonflés grâce à cette ventilation. L'injection du fluide médicamenteux touche donc la totalité de la surface desdits poumons, de sorte que ledit fluide peut avoir l'efficacité maximale.

De préférence, ledit orifice de distribution de fluide médicamenteux est relié à l'extrémité proximale dudit tube par un canal, qui est prévu dans l'épaisseur dudit tube et par l'intermédiaire duquel il est alimenté en fluide médicamenteux.

Dans le cas où, pour éviter l'usage d'une source de gaz respirable à pression élevée, le dispositif selon la présente invention comporte, comme cela est décrit dans le document FR-A-2 782 925, une bague disposée dans ledit canal principal en aval (par rapport audit jet de gaz respirable) desdits moyens de déflexion et entourant ladite zone de pression oblongue en obturant au moins partiellement l'espace périphérique dudit canal principal compris entre ladite paroi interne de celui-ci et ladite zone de pression oblongue, il est avantageux que ladite bague soit disposée entre ledit orifice de distribution et lesdits moyens de déflexion.

Par ailleurs, afin d'augmenter encore l'efficacité du dispositif conforme à la présente invention, il est préférable que l'alimentation dudit orifice de distribution en fluide médicamenteux soit interrompue pendant les expirations du patient, de sorte que ledit fluide médicamenteux n'est introduit dans le poumon de ce dernier que pendant ses inspirations.

Les figures du dessin annexé feront bien comprendre comment l'invention peut être réalisée. Sur ces figures, des références identiques désignent des éléments semblables.

La figure 1 est une vue schématique et partielle, en coupe axiale agrandie, d'un mode de réalisation du dispositif de l'invention.

Les figures 2 et 3 sont des coupes transversales, respectivement selon les lignes II-II et III-III de la figure 1.

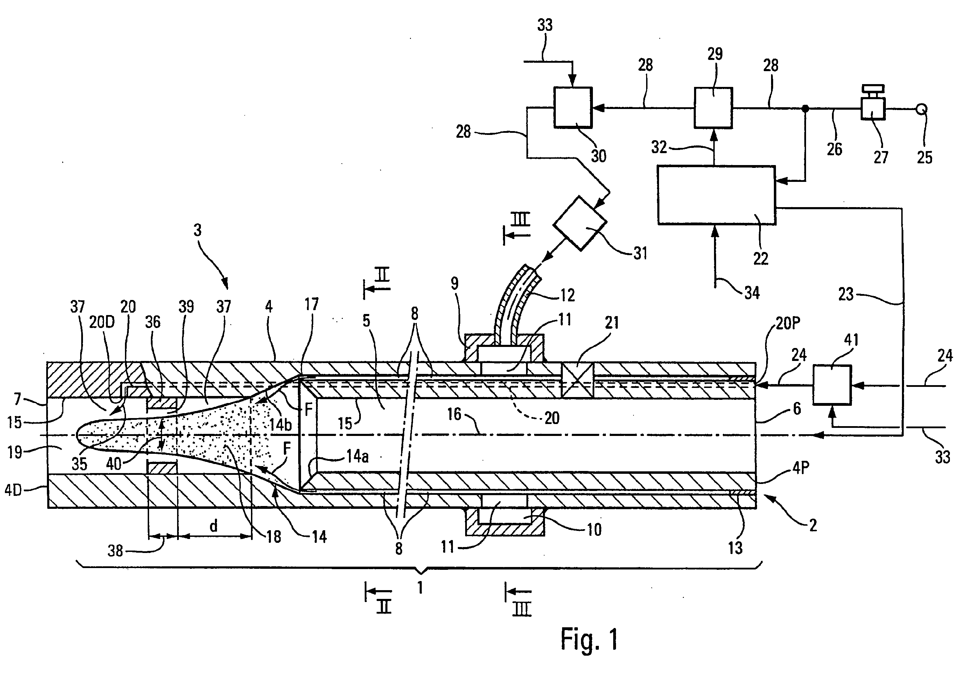

Sur la figure 1, on a représenté, schématiquement et à grande échelle, les seules extrémités proximale 2 et distale 3 d'un mode de réalisation 1 du dispositif selon l'invention. Ce mode de réalisation peut constituer, par exemple, une sonde endotrachéale oro-nasale avec ou sans ballonnet, une sonde endotrachéale pédiatrique, une sonde de monitorage des gaz, une sonde endobronchique, une sonde naso-pharyngée, une sonde d'intubation anatomique pour enfant, une sonde de Cole néonatale, une sonde canule de Gedel, une sonde nasale d'oxygénothérapie, un masque nasal ou bucconasal ou un ballon nasal pour traitement d'apnée du sommeil.

Le dispositif 1 comporte un tube 4, souple ou préformé (pour s'adapter à la morphologie du patient) délimitant un canal principal 5 débouchant, par l'orifice 6, à l'extrémité proximale 2 et, par l'orifice 7, à l'extrémité distale 3.

Ainsi, le canal principal 5 est capable d'assurer le passage entre les orifices 6 et 7, dont l'un (l'orifice distal 7) est destiné à se trouver à l'intérieur des voies respiratoires d'un patient, et l'autre (l'orifice proximal 6) est destiné à se trouver à l'extérieur dudit patient. Cet orifice proximal 6 peut déboucher à l'air libre et, dans ce cas, le patient peut inspirer de l'air frais et expirer l'air vicié à travers le canal principal 5. On peut également, comme cela est expliqué ci-après, relier l'orifice 6 à une source de gaz respirable sous pression et prévoir un système de valves unidirectionnelles, pour que le patient inspire le gaz respirable de ladite source à travers ledit canal principal 5 et expire le gaz vicié à l'air libre, également à travers ce canal principal.

Le diamètre du canal principal 5 est de l'ordre de quelques millimètres. Des essais satisfaisants ont été effectués avec des diamètres de 3 mm, 7 mm, 8 mm et 12 mm.

Par ailleurs, dans l'épaisseur de la paroi du tube 4, sont ménagés des canaux auxiliaires 8, s'étendant sur la presque totalité de la longueur du canal principal 5 et destinés à être reliés à une source de gaz respirable sous pression, comme cela est décrit ci-après.

La liaison à la source de gaz respirable sous pression peut être réalisée au moyen d'une bague 9, entourant de façon étanche le tube 4, du côté de l'extrémité proximale 2, et délimitant une chambre annulaire étanche 10 autour dudit tube. Les canaux auxiliaires 8 sont mis en communication avec la chambre 10, grâce à des arrachements locaux 11 de la paroi du tube 4, et ladite chambre 10 est reliée à ladite source de gaz respirable par un conduit 12. Bien entendu, les extrémités proximales des canaux 8 sont obturées, par exemple par des bouchons 13, introduits à partir de la face d'extrémité proximale 4P du tube 4.

Les canaux auxiliaires 8 ont un diamètre plus petit que celui du canal principal 5. Le diamètre des canaux auxiliaires 8 est de préférence inférieur à 1 mm et, de façon avantageuse, il est de l'ordre de 400 à 800 microns. Du côté distal, les canaux auxiliaires 8 débouchent dans un évidement 14 de la paroi interne 15 du tube 4. L'évidement 14 est annulaire et centré sur l'axe 16 de l'extrémité distale 3. Il comporte une face 14a, sensiblement transversale ou légèrement inclinée de façon à constituer un évasement du canal principal 5, dans laquelle débouchent lesdits canaux auxiliaires 8 par leurs orifices 17, ainsi qu'une face 14b suivant la face 14a et convergeant en direction de l'axe 16.

Ainsi, lorsque les canaux auxiliaires 8 sont alimentés en gaz respirable sous pression à travers les éléments 9 à 12, les jets gazeux correspondants heurtent la face inclinée 14b, qui les défléchit en direction de l'axe 16 (flèches F sur la figure 1), engendrant à l'intérieur de l'extrémité distale 3 du canal principal 5 une zone de pression 18 de forme oblongue prenant naissance auxdits orifices distaux 17 et s'allongeant en direction de l'orifice distal 7 le long de l'axe 16 de ladite extrémité distale 3. La section transversale de la zone de pression 18 décroît progressivement depuis l'évidement 14 vers l'orifice distal 7, ladite zone de pression 18 s'écartant progressivement de la paroi interne 15 du tube 4 pour n'occuper que la partie centrale de l'extrémité distale 3 de ce dernier. En aval de la zone de pression 18, les jets de gaz respirable défléchis engendrent au voisinage de l'axe 16 une zone de dépression 19 favorisant la circulation gazeuse à l'intérieur du canal principal 5, de l'orifice proximal vers l'orifice distal. On favorise ainsi l'inspiration du patient.

Au moins un canal supplémentaire 20 est prévu dans l'épaisseur du tube 4. Ce canal supplémentaire 20 comporte un orifice proximal 20P débouchant dans la face d'extrémité proximale 4P dudit tube 4, et un orifice distal 20D, pratiqué dans la paroi interne 15 dudit tube 4 et débouchant dans le canal principal 5, en regard de la zone de pression 18. Le canal supplémentaire 20 peut être alimenté en fluide médical sous pression --gaz ou liquide-- à travers son orifice proximal 20P, par une conduite 24, reliée à une source (non représentée) d'un tel fluide. Sur la conduite 24 est montée une vanne commandable 41.

A titre de sécurité, une soupape d'échappement tarée 21 peut être prévue dans l'extrémité proximale2 du tube 4. Ainsi, en cas de surpression accidentelle dans le canal principal 5, une fuite de gaz se produit à l'extérieur du patient, à travers la paroi du tube 4, pour éliminer instantanément cette surpression.

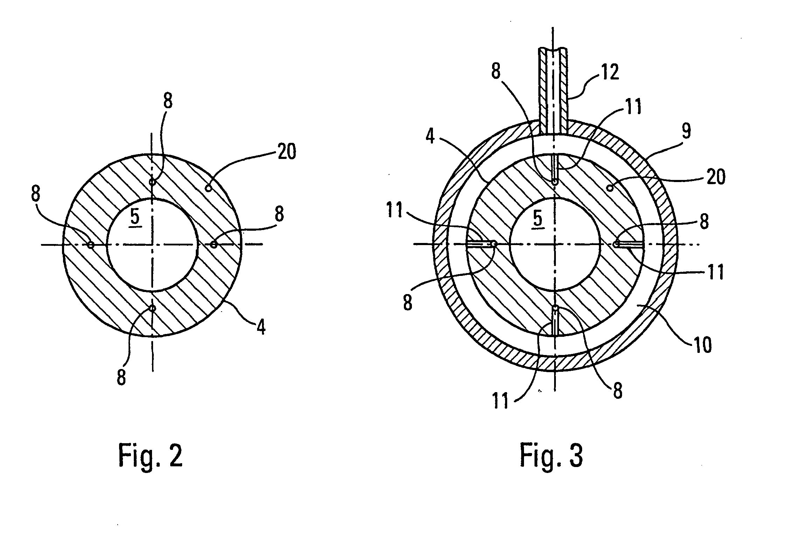

Comme le montrent les figures 2 et 3, les canaux auxiliaires 8 sont disposés régulièrement autour de l'axe du tube 4. Leur nombre est variable suivant les utilisations (adulte ou enfant), mais il est généralement compris entre trois et neuf.

Le tube 4 du dispositif selon l'invention peut être réalisé en toute matière déjà utilisée dans les sondes respiratoires, par exemple en un chlorure de polyvinyle, avec un éventuel revêtement de silicone ou en acier permettant les injections à pression élevée.

Bien entendu, les dimensions du dispositif selon l'invention peuvent être très variables, essentiellement en fonction de la voie de mise en place du tube et de la taille du patient, qui peut être un adulte, un enfant, un nourrisson ou un prématuré.

Le dispositif 1 comporte de plus un dispositif d'alimentation et de commande 22, qui est relié à l'orifice proximal 6 du tube 4 par une liaison 23 et qui peut être commandé par un capteur de pression (non représenté) par l'intermédiaire d'une liaison 34. La prise de pression de ce dernier capteur peut être disposée dans la face distale 4D.

Le dispositif d'alimentation et de commande 22 est alimenté en gaz respirable sous pression par une source 25, à laquelle il est relié par une conduite 26 sur laquelle est monté un détendeur-débitmètre réglable 27.

La sortie du détendeur-débitmètre 27 est reliée au conduit 12 par une conduite de dérivation 28 sur laquelle sont montés en série une première vanne commandable 29, une seconde vanne commandable 30 et un humidificateur 31.

La vanne commandable 29 est commandée par le dispositif d'alimentation et de commande 22 par l'intermédiaire d'une liaison 32.

La vanne commandable 30 est commandée, par l'intermédiaire d'une liaison 33, par un capteur de pression (non représenté) apte à détecter les passages de l'inspiration à l'expiration dans la respiration du patient. La prise de mesure de ce capteur de pression peut se trouver au voisinage de l'orifice distal 7. La liaison 33 commande également la vanne commandable 41 d'alimentation en fluide médicamenteux.

Dans l'extrémité distale 3, peut être disposée une bague 36 entourant la zone de pression centrale 18 et occupant localement au moins en partie l'espace périphérique annulaire 37 compris entre ladite zone de pression centrale 18 et la paroi interne 15 de l'extrémité distale 3 du canal 5.

Grâce à une telle bague 36, la pression de la source de gaz respirable 25, nécessaire à l'obtention de la zone de pression 18, peut être abaissée.

En règle générale, la distance d entre la bague 36 et la face inclinée de déflexion 14b est voisine du diamètre de la partie distale du canal principal 5.

Pour permettre d'obtenir la réduction optimale de pression nécessaire de la source 25, cette distance d peut être réglable, comme cela est illustré par la double flèche 38. Il est également avantageux, à la même fin, que le diamètre de l'ouverture centrale 39 de la bague 36 soit réglable, comme cela est illustré par la double flèche 40. Ce double réglage peut être obtenu grâce à un jeu de plusieurs bagues interchangeables 36 pouvant, au choix, être montées en coulissement dans l'extrémité distale du canal principal 5. La bague 36 peut, en variante, être constituée par un anneau gonflable, dont on peut faire varier le diamètre intérieur par gonflage.

Les modes de fonctionnement du dispositif 1 selon l'invention sont les suivants :
- dans le mode de respiration artificielle, le dispositif d'alimentation et de commande 22, d'une part, commande la vanne 29 à la fermeture par l'intermédiaire de la liaison 32, de sorte que le conduit 12 n'est pas alimenté en gaz et, d'autre part, adresse du gaz respirable dans le tube 4 par l'intermédiaire de la liaison 23. Ce dispositif 22 comporte des moyens (non représentés) pour permettre le réglage de la pression et du débit de gaz respirable qu'il reçoit de la conduite 26 et qu'il adresse au tube 4. Si une surpression se produit dans la voie respiratoire du patient, elle est détectée et transmise par la liaison 34 au dispositif 22 qui arrête son fonctionnement. De plus, si cette surpression dépasse le seuil de tarage de la soupape tarée 21, celle-ci s'ouvre et le canal proximal 5 est mis à l'atmosphère ;
- dans le mode d'assistance respiratoire, le dispositif d'alimentation et de commande 22 coupe la liaison 23 pour mettre l'orifice proximal 6 en communication avec l'atmosphère et commande la vanne 29 par la liaison 32 pour que cette vanne adresse au patient des jets, continuels ou impulsionnels, de gaz respirable à travers la vanne 30, l'humidificateur 31 et les canaux auxiliaires 8.
Il se forme alors la zone de pression 18 et la zone de dépression 19 permettant la ventilation aisée du patient. Celui-ci peut donc inspirer librement et profondément. Simultanément, la vanne 41 étant ouverte, du fluide médicamenteux est introduit dans les poumons du patient, à travers la conduite 24, le canal 20 et l'orifice 20D.
Lorsqu'après une inspiration, le patient commence à expirer, ce début d'expiration est détecté et les vannes 30 et 41 sont commandées à la fermeture par la liaison 33. Les jets de gaz respirables, la zone de pression 18, la zone de dépression 19 et l'alimentation en fluide médicamenteux disparaissent donc et le patient peut librement expirer à travers le canal principal 5.
Si, pendant la ventilation, une surpression se produit dans la voie respiratoire du patient, comme cela a été décrit ci-dessus, cette surpression est détectée et transmise par le canal supplémentaire 20, de sorte que le dispositif 22 ferme la vanne 29 et que la ventilation est arrêtée.

De ce qui précède, on voit donc que, grâce à l'invention, on obtient, avec une source de pression modérée, une assistance respiratoire efficace et sûre, humidifiée et accompagnée d'une injection de fluide médicamenteux permettant la disparition quasiment totale de l'espace mort inhérent aux sondes connues. De plus, on notera que le dispositif d'assistance respiratoire peut être réalisé pour être léger et transportable, afin de pouvoir être utilisé en urgence, à l'extérieur d'un hôpital ou d'une clinique.

## Revendications

1. Dispositif d'assistance respiratoire comportant un tube (4) qui forme un canal principal (5) et qui est destiné à être relié par son extrémité distale (3) à une voie respiratoire d'un patient pour que ledit canal principal (5) relie, à l'extérieur, le système respiratoire dudit patient, ledit dispositif comportant de plus au moins un canal auxiliaire (8) qui est relié, à son extrémité proximale, à une source de gaz respirable (25) pour pouvoir insuffler un jet d'un tel gaz respirable dans ledit système respiratoire et dont l'extrémité distale débouche dans ledit canal principal (5) au voisinage de l'extrémité distale (7) de ce dernier, des moyens (14b) de déflexion dudit jet de gaz respirable de ventilation en direction de l'axe (16) dudit canal principal (5) étant prévus en regard de l'orifice distal (17) dudit canal auxiliaire (8), de sorte que, en aval desdits moyens de déflexion, il se forme, à l'intérieur dudit canal principal, une zone de pression (18) de forme oblongue prenant naissance audit orifice distal (17) et.s'allongeant dans la direction distale le long de l'axe (16) dudit canal principal (5) avec réduction progressive de sa section transversale en s'écartant de la paroi interne (15) dudit canal principal pour n'occuper que la partie centrale de ce dernier, ledit tube (4) comportant en aval desdits moyens de déflexion (14b), au moins un orifice qui débouche dans ledit canal principal (5) en regard de ladite zone de pression oblongue (18),
**caractérisé en ce qu**'il comporte une vanne commandable (41) pour alimenter ledit orifice (20D) en fluide médicamenteux ladite vanne commandable est contrôlable pour l'ouverture pendant les inspirations dudit patient et contrôlable pour la fermeture pendant les expirations de celui-ci et ledit orifice (20D) étant apte à distribuer ledit fluide médicamenteux dans ledit tube (4).

2. Dispositif selon la revendication 1,
**caractérisé en ce que** ledit orifice de distribution (20D) est relié à l'extrémité proximale (2) dudit tube (4) par un canal (20), qui est prévu dans l'épaisseur dudit tube (4) et par l'intermédiaire duquel il est alimenté en fluide médicamenteux.

3. Dispositif selon l'une des revendications 1 ou 2, comportant une bague (36) disposée dans ledit canal principal (5) en aval desdits moyens de déflexion (14b) et entourant ladite zone de pression oblongue (18) en obturant au moins partiellement l'espace périphérique (37) dudit canal principal compris entre ladite paroi interne (15) de celui-ci et ladite zone de pression oblongue (18),
**caractérisé en ce que** ladite bague (36) est disposée entre ledit orifice de distribution (20D) et lesdits moyens de déflexion (14b).

## Claims

1. A device for respiratory assistance comprising a tube (4) which forms a main channel (5) and which is intended to be connected via its distal end (3) to an airway of a patient so that said main channel connects said patient's respiratory system to the outside, said device further comprising at least one auxiliary channel (8) which is connected, at its proximal end, to a source of respirable gas (25) in order to be able to insufflate a jet of this respirable gas into said respiratory system and whose distal end opens into said main channel (5) in proximity to the distal end (7) of the latter, deflection means (14b) for deflecting said jet of respirable ventilation gas in the direction of the axis (16) of said main channel (5) being provided opposite the distal orifice (17) of said auxiliary channel (8) in such a way that, downstream of said deflection means, a pressure zone (18) of oblong shape forms inside said main channel, starting at said distal orifice (17) and continuing in the distal direction along the axis (16) of said main channel (5), with progressive reduction of its cross section as it moves away from the inner wall (15) of said main channel and comes to occupy only the central part of the latter, said tube (4) comprising, downstream of said deflection means (14b), at least one orifice which opens into said main channel (5) opposite said oblong pressure zone (18),
**characterized in that** said device comprises a controllable valve (41) to supply said orifice (20D) with a medical fluid, said controllable valve being open-controlled during the inhalations of the patient and closed-controlled during the exhalations of this latter, and said orifice (20D) being able to supply said tube (4) with said medical fluide.

2. The device as claimed in claim 1,
**characterized in that** said distribution orifice (20D) is connected to the proximal end (2) of said tube (4) via a channel (20) which is provided within the thickness of said tube (4) and by way of which it is supplied with medical fluid.

3. The device as claimed in one of claims 1 or 2, comprising a ring (36) arranged in said main channel (5) downstream of said deflection means (14b) and surrounding said oblong pressure zone (18), at least partially obturating the peripheral space (37) of said main channel located between said inner wall (15) of the latter and said oblong pressure zone (18),
**characterized in that** said ring (36) is arranged between said distribution orifice (20D) and said deflection means (14b).

## Patentansprüche

1. Vorrichtung zur Atmungsunterstützung, umfassend einen Tubus (4), der einen Hauptkanal (5) bildet und der dazu bestimmt ist, durch sein distales Ende (3) mit einem Atemweg eines Patienten verbunden zu werden, damit der Hauptkanal (5) das Atmungssystem des Patienten mit der Außenseite verbindet, wobei die Vorrichtung darüber hinaus mindestens einen zusätzlichen Kanal (8) umfasst, der an seinem proximalen Ende mit einer Atemgasquelle (25) verbunden ist, um einen Strahl eines solchen Atemgases in das Atmungssystem einblasen zu können, und dessen distales Ende in der Nähe des distalen Endes (7) des Hauptkanals in den Hauptkanal (5) mündet, wobei Mittel (14b) zur Ablenkung des zur Beatmung dienenden Atemgasstrahls in Richtung der Achse (16) des Hauptkanals (5) am Ort der distalen Öffnung (17) des zusätzlichen Kanals (8) vorgesehen sind, so dass sich stromabwärts von den Ablenkungsmittel im Inneren des Hauptkanals eine Druckzone (18) von langgestreckter Form bildet, die an der distalen Öffnung (17) ihren Ursprung hat und sich in distaler Richtung entlang der Achse (16) des Hauptkanals (5) mit zunehmender Verringerung ihres Querschnitts erstreckt, wobei sie sich von der Innenwand (15) des Hauptkanals erstreckt, um nur mehr den mittleren Teil des Letztgenannten einzunehmen, wobei der Tubus (4) stromabwärts von den Ablenkungsmitteln (14b) mindestens eine Öffnung umfasst, die am Ort der langgestreckten Druckzone (18) in den Hauptkanal (5) mündet,
**dadurch gekennzeichnet, dass** sie ein steuerbares Ventil (41) umfasst, um die Öffnung (20D) mit einem Medikamentenfluid zu versorgen, wobei das steuerbare Ventil während des Einatmens des Patienten zum Zweck der Öffnung steuerbar ist und während des Ausatmens des Letztgenannten zum Zweck des Schließens steuerbar ist, und wobei die Öffnung (20D) geeignet ist, das Medikamentenfluid in dem Tubus (4) zu verteilen.

2. Vorrichtung nach Anspruch 1,
**dadurch gekennzeichnet, dass** die Verteilungsöffnung (20D) mit dem proximalen Ende (2) des Tubus (4) durch einen Kanal (20) verbunden ist, der in der Dicke des Tubus (4) vorgesehen ist und über den sie mit dem Medikamentenfluid versorgt wird.

3. Vorrichtung nach einem der Ansprüche 1 oder 2, umfassend einen Ring (36), der in dem Hauptkanal (5) stromabwärts von den Ablenkungsmitteln (14b) angeordnet ist und die langgestreckte Druckzone (18) umgibt, wobei er mindestens teilweise den Umfangsraum (37) des Hauptkanals zwischen der Innenwand (15) des Letztgenannten und der langgestreckten Druckzone (18) verschließt,
**dadurch gekennzeichnet, dass** der Ring (36) zwischen der Verteilungsöffnung (20D) und den Ablenkungsmitteln (14b) angeordnet ist.
